# EUROPEAN PATENT APPLICATION

(11) **EP 2 345 713 A1**
(43) Date of publication of application: **20.07.2011**
(21) Application number: 08734108.7
(22) Date of filing: 18.04.2008
(51) Int. Cl.: C12N 5/06, C12N 5/16, C12R 1/91

(54) **A CONCENTRATED CULTURE SOLUTION AND APPLICATION METHOD THEREOF**

(71) Applicant: Shanghai CP Guojian Pharmaceutical Co. Ltd, Shanghai 201203 (CN)
(72) Inventor: GUO, Yajun, Shanghai 201203 (CN); WANG, Hao, Shanghai 201203 (CN); KOU, Geng, Shanghai 201203 (CN); HU, Hui, Shanghai 201203 (CN); HOU, Sheng, Shanghai 201203 (CN); TAN, Min, Shanghai 201203 (CN)
(74) Representative: Martin Santos, Victoria Sofia
(86) International application number: PCT/CN2008/070750
(87) International publication number: WO 2009/127098

(57) **Abstract**

This invention discloses a kind of concentrated medium for large-scale culture of Chinese Hamster Ovary cell. The said medium comprises basic medium and high dose additives. This invention also discloses the method of adding the described concentrated medium in large-scale fed-batch culture of CHO cells.

## Description

### FIELD OF THE INVENTION:

This invention relates to cell culture. More specifically, this invention relates to a kind of concentrated medium which is suitable for animal cell culture on large-scale, and its usage.

### BACKGROUND OF THE INVENTION AND PRIOR ART:

Animal cells culture on large-scale has been widely used in producing all kinds of biologically active substances, such as monoclonal antibodies, vaccines, immune regulatory factors, growth factors, tumor-specific antigens and various gene-recombinant protein drugs. Compared with microorganisms, animal cells have capacity of post-transcriptional modification which can efficiently express and produce various high-qualified proteins.

No matter adherent or suspension animal cell, the culture mode is mainly divided into three kinds: batch, fed-batch and perfusion. At present, fed-batch culture has been considered as the most popular mode in large-scale animal cell culture on production of secretary recombinant protein drugs.

In all the animal cells, nowadays Chinese hamster ovary (CHO) cell has been widely used as host cell for producing various kinds of genetically engineered protein products included monoclonal antibodies, fusion protein, vaccines, cytokines, tumor-specific antigens and various gene-recombinant protein drugs. The development of medium suitable for the cell culture can be divided into three stages: natural medium stage, synthetic medium stage, and serum-free medium stage according to the clarity of its source and composition. The development of serum-free medium at home and abroad is involved in many aspects. Among them, a kind of "domestic improved DMEM medium" was used to culture CHO-C28 cells (disclosed by Yao Wei in « Chinese Journal of Biologicals » 2003 , 16 ( 6 ) : 380-383 ) . It solved the problems as easy thickening, shedding and difficulty to maintain through the course of large scale culture of CHO-C28 cell. A kind of "purification human CD34⁺ cell of serum-free animal culture" was disclosed by Zhao Guosheng in « International Journal of Blood Transfusion and Hematology Volumes » ) 1998 , 21(4):263-264), such animal serum-free(ASF) medium was introduced in culturing of CD34⁺ cells isolated from human bone marrow and peripheral blood. In this system, combination of different growth factors were added in the ASF for CD34⁺ cells culture. Compared with the medium containing 10% fetal calf serum, ASF is also suitable for stem cells and progenitors growth well. CN 00816020 ( title of the invention: medium with protein-free serum-free for culturing cell ) disclosed a kind of protein-free and serum-free medium used to cell culture, especially to mammalian cells, in which contains a certain proportion of soybean hydrolysate. WO200123527 also disclosed a kind of protein-free and serum-free medium that contains soybean hydrolyzate, said medium comprises soybean hydrolyzate less than 10% of the total dry weight, endotoxin less than 500U/g, said medium also contains amino acids (cysteine, proline, and tryptophan) or amino acids mixture, it also contains other auxiliary components, buffering factors, antioxidants and protease inhibitors etc. There is another kind of serum-free medium without animal component which is suitable for CHO cells culture on large-scale. It solves the problems as low cell density and low protein expression in the large-scale cell culture process.

A kind of medium suitable for CHO cell culture on large-scale both with serum and animal component free was disclosed in CN 200710085142.2, It solved the problem of low cell density and low protein expression in the sell culture process on large-scale. The medium mentioned above had been applied to the process of continuous perfusion culture and obtained good results. But in the process of fed-batch, the key problem of the culture result focuses on the supplement of nutrition components in the later stage of cell culture.

Therefore, a kind of medium which contains nutrition components and can be supplemented in the later stage of cell culture is needed urgently in this field. This kind of medium is essential to the result of cell culture in the process of fed-batch.

### CONTENT OF THE INVENTION:

The main object of this invention is to provide a kind of concentrated medium used in culturing CHO cells on large-scale.

Another object of this invention is to provide preparation method of the said concentrated medium.

Still another object of this invention is to provide the usage of the said concentrated medium.

The first aspect of this invention provides a kind of concentrated medium used in culturing Chinese Hamster Ovary cell on large-scale. It comprises basic medium and additives, the said additives comprises the following components according to the total volume of the concentrated medium.

| | |
|---|---|
| Vitamin C | 10-25mg/L |
| Transferrin | 5-18mg/L |
| Ethanolamine | 1-10mg/L |
| Selenite | 0.01-0.045 mg/L |
| Hydroxybutyrate sodium | 0.5-1.5mg/L |
| AlCl₃•6H₂O | 0.5-2.5mg/L |
| AgNO₃ | 0.02-0.045mg/L |
| CoCl₃•6H₂O | 0.05-0.3mg/L |
| CuSO₄•5H₂O | 0.002-0.004mg/L |
| KBr | 0.0005-0.0008mg/L |
| FeSO₄•7H₂O | 0.5-1.5mg/L |
| Na₂SiO₃ | 0.005-0.045mg/L |
| LiCl | 0.0005-0.001mg/L |
| NiCl₂•6H₂O | 0.02-0.25 mg/L |
| SnCl₂•2H₂O | 2.00-8.00mg/L |
| ZnSO₄•7H₂O | 0.5-2.5mg/L |
| Vitamin B6 | 0.01-0.2mg/L |
| Vitamin B12 | 0.01-0.5mg/L |
| Biotin | 0.05-0.18mg/L |
| Folic Acid | 0.02-8.00mg/L |
| Ribo flavin | 0.1-0.15mg/L |
| reduced glutathione | 0.5-2.5mg/L |
| Adenine | 5.5-8.5 mg/L |
| Guanine | 5.5-8.5 mg/L |
| Uracil | 5.5-8.5 mg/L |
| Thymine | 0.25-1.5mg/L |
| Cytosine | 5.5-8.5mg/L |
| Ribose | 2.0-8.5mg/L |
| Deoxyribose | 2.0-8.5mg/L |
| Primatone | 500-3500mg/L |
| 4-hydroxyethyl-piperazine ethanesulfonic acid (HEPES) | 500-3000mg/L |
| β-mercaptoethanol | 0.2-2mg/L, |

the concentrations of the components of the concentrated medium as below:

| | |
|---|---|
| 1000-9000mg/L | Sodium chloride |
| 200-4000 mg/L | Aspartate |
| 200-9000 mg/L | Asparagine |
| 100-5000 mg/L | Glutamate |
| 200-3000 mg/L | Isoleucine |
| 300-4500 mg/L | Leucine |
| 50-1000 mg/L | Methionine |
| 100-2000 mg/L | Phenylalanine |
| 500-10000 mg/L | Serine |
| 50-1500 mg/L | Threonine |
| 25 -1000 mg/L | Tryptophan |
| 40-1000 mg/L | Valine |
| 150-2000 mg/L | Arginine |
| 0.01- 0.5 mg/L | Biotin |
| 2-100 mg/L | Calcium Pantothenate |
| 3-270 mg/L | Choline chloride |
| 1-30 mg/L | Folic Acid |
| 5-900 mg/L | Inositol |
| 0.1-10 mg/L | Nicotinic acid amine |
| 0.01-1 mg/L | Riboflavin |
| 1-100 mg/L | Thiamine |
| 0.01-5 mg/L | Vitamin B6. |

In another preferred embodiment, the concentrations of the components of concentrated medium as below :

| | |
|---|---|
| 1500-6599 mg/L | Sodium chloride |
| 350-2000 mg/L | Aspartate |
| 550-5000 mg/L | Asparagine |
| 250-3000 mg/L | Glutamate |
| 500-2500 mg/L | Isoleucine |
| 500-2500 mg/L | Leucine |
| 80-700 mg/L | Methionine |
| 200-1250 mg/L | Phenylalanine |
| 2000-7000 mg/L | Serine |
| 100-700 mg/L | Threonine |
| 80-350 mg/L | Tryptophan |
| 80-400 mg/L | Valine |
| 300-1200 mg/L | Arginine |
| 0.05-0.2 mg/L | Biotin |
| 10-70 mg/L | Calcium Pantothenate |
| 10-120 mg/L | Choline chloride |
| 2-15 mg/L | Folic Acid |
| 25-500 mg/L | Inositol |
| 0.5-5 mg/L | Nicotinic acid amine |
| 0.1-0.5 mg/L | Riboflavin |
| 5-50 mg/L | Thiamine |
| 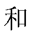 0.1-2 mg/L | Vitamin B6 |

In another preferred embodiment, the concentrations of the components of concentrated medium as below :

| | |
|---|---|
| 2000-4500 mg/L | Sodium chloride |
| 450-1200 mg/L | Aspartate |
| 1000-4500 mg/L | Asparagine |
| 700-2200 mg/L | Glutamate |
| 900-1800 mg/L | Isoleucine |
| 700-1400 mg/L | Leucine |
| 300-500 mg/L | Methionine |
| 250-500 mg/L | Phenylalanine |
| 3000-5500 mg/L | Serine |
| 250-500 mg/L | Threonine |
| 90-270 mg/L | Tryptophan |
| 120-350 mg/L | Valine |
| 400-700 mg/L | Arginine |
| 0.07-0.12 mg/L | Biotin |
| 20-60 mg/L | Calcium Pantothenate |
| 20-90 mg/L | Choline chloride |
| 3-12 mg/L | Folic Acid |
| 65-300 mg/L | Inositol |
| 0.8-3 mg/L | Nicotinic acid amine |
| 0.12-0.3 mg/L | Riboflavin |
| 10-35 mg/L | Thiamine |
| 0.2-1.5 mg/L | Vitamin B6 |

In another preferred embodiment, the said basic medium is Dulbecco's Modified Eagle's medium (DMEM ) .

The second aspect of this invention provides the preparation method of the concentrated medium. The said method comprises steps below: mixing the basic medium and additives. The additives mentioned above comprises following components( according to the total volume of the concentrated medium):

| | |
|---|---|
| Vitamin C | 10-25mg/L |
| Transferrin | 5-18mg/L |
| Ethanolamine | 1-10mg/L |
| Selenite | 0.01-0.045 mg/L |
| Hydroxybutyrate sodium | 0.5 -1.5mg/L |
| AlCl₃•6H₂O | 0.5-2.5mg/L |
| AgNO₃ | 0.02-0.045mg/L |
| CoCl₃•6H₂O | 0.05 -0.3mg/L |
| CuSO₄•5H₂O | 0.002-0.004mg/L |
| KBr | 0.0005-0.0008mg/L |
| FeSO₄•7H₂O | 0.5-1.5mg/L |
| Na₂SiO₃ | 0.005-0.045mg/L |
| LiCl | 0.0005-0.001mg/L |
| NiCl₂•6H₂O | 0.02-0.25 mg/L |
| SnCl₂•2H₂O | 2.00-8.00mg/L |
| ZnSO₄•7H₂O | 0.5-2.5mg/L |
| Vitamin B6 | 0.01-0.2mg/L |
| Vitamin B12 | 0.01-0.5mg/L |
| biotin | 0.05-0.18mg/L |
| Folic acid | 0.02-8.00mg/L |
| Ribo flavin | 0.1-0.15mg/L |
| Reduced Glutathione | 0.5-2.5mg/L |
| Adenine | 5.5-8.5 mg/L |
| Guanine | 5.5-8.5 mg/L |
| Uracil | 5.5-8.5 mg/L |
| Thymine | 0.25-1.5mg/L |
| Cytosine | 5.5-8.5mg/L |
| Ribose | 2.0-8.5mg/L |
| Deoxyribose | 2.0-8.5mg/L |
| Primatone | 500-3500mg/L |
| 4-hydroxyethyl-piperazine ethanesulfonic acid ((HEPES) | 500-3000mg/L |
| β- mercaptoethanol | 0.2-2mg/L, |

The concentrations of the composition are adjusted as below:

| | |
|---|---|
| 1000-9000mg/L | Sodium chloride |
| 200-4000 mg/L | Aspartate |
| 200-9000 mg/L | Asparagine |
| 100-5000 mg/L | Glutamate |
| 200-3000 mg/L | Isoleucine |
| 300-4500 mg/L | Leucine |
| 50-1000 mg/L | Methionine |
| 100-2000 mg/L | Phenylalanine |
| 500-10000 mg/L | Serine |
| 50-1500 mg/L | Threonine |
| 25 -1000 mg/L | Tryptophan |
| 40-1000 mg/L | Valine |
| 150-2000 mg/L | Arginine |
| 0.01-0.5 mg/L | Biotin |
| 2-100 mg/L | Calcium Pantothenate |
| 3-270 mg/L | Choline chloride |
| 1-30 mg/L | Folic Acid |
| 5-900 mg/L | Inositol |
| 0.1-10 mg/L | Nicotinic acid amine |
| 0.01-1 mg/L | Riboflavin |
| 1-100 mg/L | Thiamine |
| and 0.01-5 mg/L | Vitamin B6 |

preferably , the said basic medium is Dulbecco's Modified Eagle's medium ( DMEM ) .

The third aspect of this invention provides the usage of the above-mentioned concentrated medium, which comprises the following steps:
1-5 days after cells inoculation, the above-mentioned concentrated medium is added. The adding volume is 10-100% of the initial culture volume. The said cells are Chinese Hamster Ovary (CHO) cells.
In another preferred embodiment, the said cell inoculation density is 2×10⁵/ml - 2×10⁶/ml.
In another preferred embodiment, the said CHO cells are selected from CHO cells that express sTNFR fusion protein, CHO cells that express sCTLA4 fusion protein, or CHO cells that express humanized anti-HER2 monoclonal antibody.

In another preferred embodiment, the total amount added is 15 - 60 % of the initial culture volume.

In another preferred embodiment, the rate of adding the concentrated medium is 1-5 times each day or once in 1-5 days; preferably adding rate is 2-4 times each day or once in 2-4 days.

In another preferred embodiment, the time for adding the concentrated medium last for 5-20days, preferably 8-16days.

In another preferred embodiment, volume of concentrated medium added is the same each time. Or the adding method may be as follow: adding 15% - 25% of the concentrated medium needed by volume in the first 5 days, adding 50% - 70% of the concentrated medium needed by volume in the intermediate 3-9 and adding 15% - 25% of the concentrated medium needed by volume n the last 1-5 days.

Accordingly, this invention provides a kind of medium that comprises nutrients and can be supplemented in the later part of cell culture. This kind of medium is very important for the cell culture result of the fed-batch culture process.

### BRIEF DESCRIPTION OF THE DRAWINGS

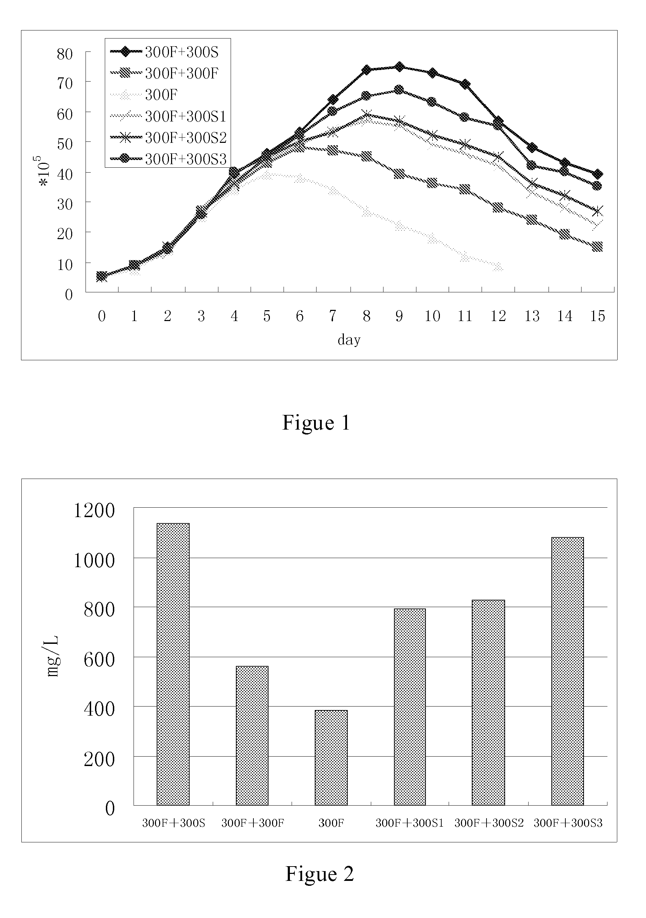

Figure 1 : Cell growth curves of fed-batch culture after adding different fed-liquid in the 5L bioreactors. There were five experiment groups (300F, 300S, 300S1, 300S2 or 300S3) and one blank control, in experiment groups, the fed medium was added by volume as 2% of the initial culture volume.
Figure 2: The expression result of cell fed-batch culture after adding different fed-liquid in the 5L bioreactors. There were five experiment groups (300F, 300S, 300S1, 300S2 or 300S3) and one blank control, in experiment groups, the fed medium was added by volume as 2% of the initial culture volume

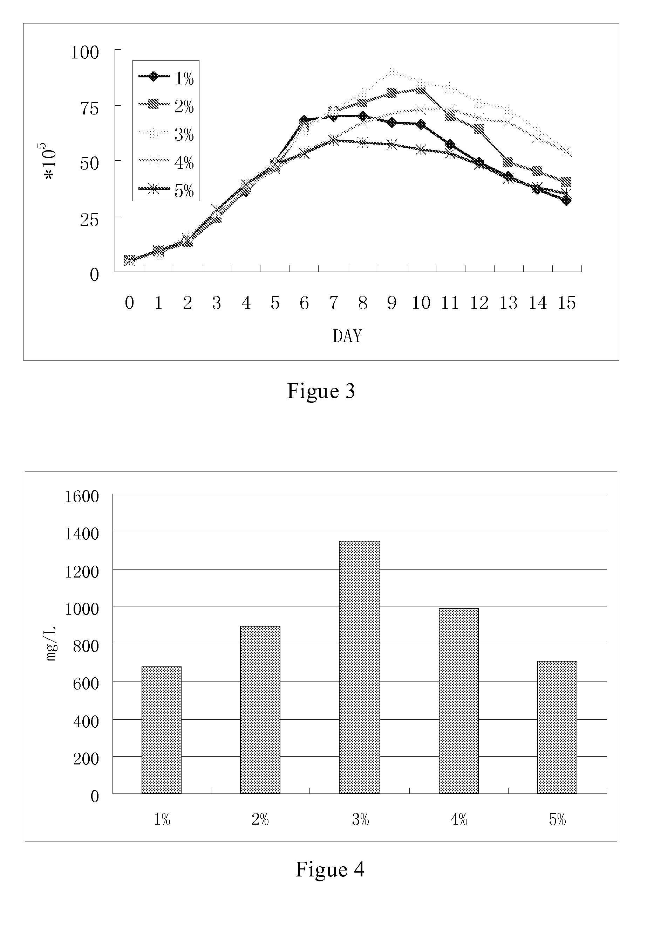

Figure 3: Cell growth curve of fed-batch culture after adding different volume of 300S in 5L bioreactors, the daily volume added was 1%, 2%, 3%, 4% or 5% of the initial culture volume.
Figure 4: The expression outcome of cell fed-batch culture after adding different volume of 300S in 5L cans, the daily volume added was 1%, 2%, 3%, 4% or 5% of the initial culture volume.

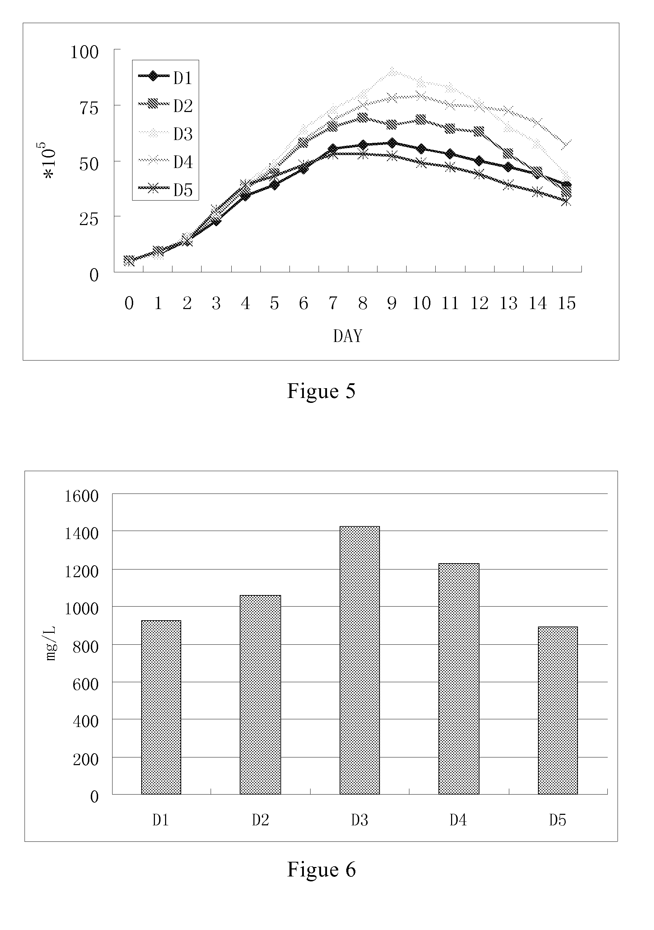

Figure 5: Cell growth curve of fed-batch culture after adding 300S started at different time in 5L bioreactors, the adding began at 1,2,3,4 or 5days after inoculation, and daily adding volume was 3% of initial culture volume.
Figure6 : The expression outcome of cell fed-batch culture after adding 300S started at different time in 5L bioreactors, the adding began at 1, 2, 3, 4 or 5days after inoculation, and daily adding volume was 3% of initial culture volume.

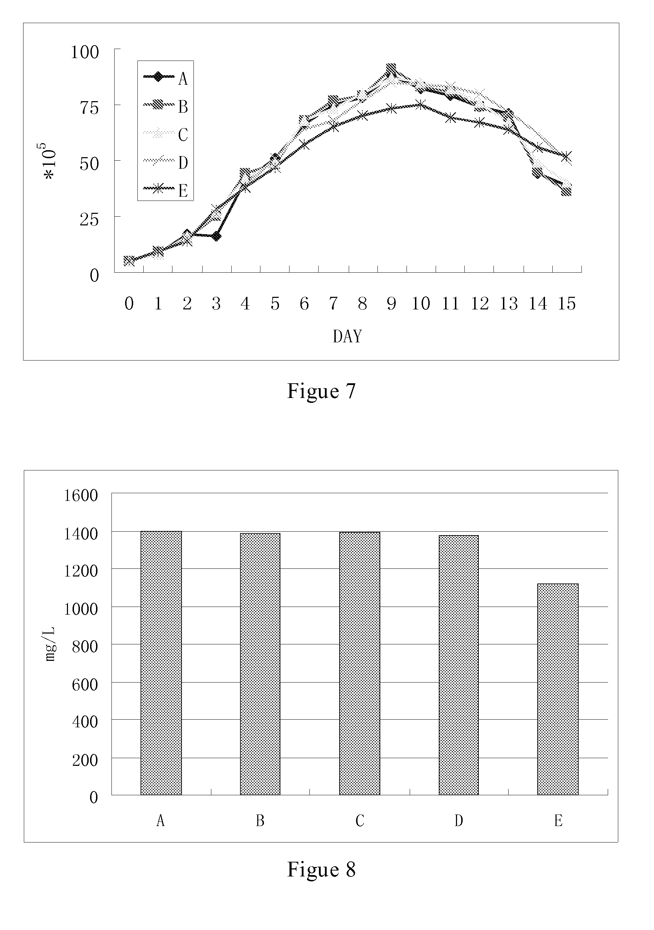

Figure 7: Cell growth curve of fed-batch culture after adding 300S with different frequencies in 5L bioreactors, the total volume added was 36% of the initial culture volume, and the volume added was same for each time. A: twice every day; B: once every day; C: once every two days; D: once every three days; E: once every four days.
Figure8 : The expression result of cell fed-batch culture after adding 300S with different frequencies in 5L bioreactors, the total volume added was 36% of the initial culture volume, and the volume added was same for each time. A: twice every day; B: once every day; C: once every two days; D once every three days; E: once every four days.

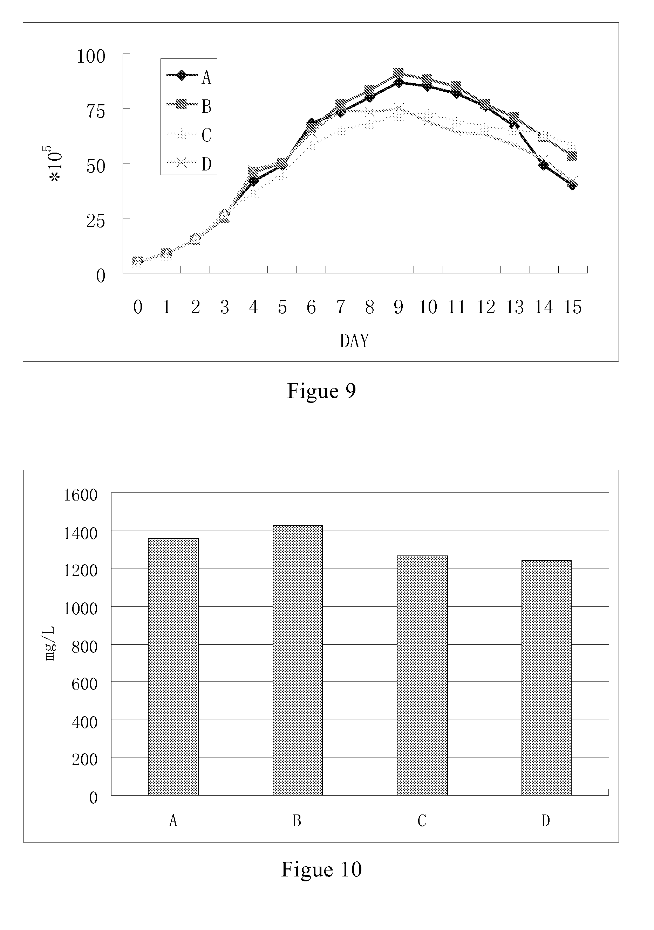

Figure9 : Cell growth curves of fed-batch culture after adding 300S by different schemes in 5L bioreactors, the total volume added was 36% of the initial culture volume, and the volume added was different for each time,the fed medium was added at 3rd, 6th, 9th, 12th days after inoculation,the adding volume was: A : 9%(3rd d) - 9%(6^{th} d) - 9%(9^{th}d) - 9%(12^{th} d) ; B : 6%(3rd d) - 12%(6^{th} d) - 12%(9^{th}d) - 6%(12^{th} d) ; C : 12%(3rd d) - 12%(6^{th} d) - 6%(9^{th}d) - 6%(12^{th} d) ; D : 6%(3rd d ) - 6%(6^{th} d) - 12%(9^{th}d) - 12%(12^{th} d) .
Figure 10 : The expression result of cell fed-batch culture after added 300S by different scheme in 5L bioreactors, the total volume added was 36% of the initial culture volume, and the volume added was different for each time, the fed medium was added at 3rd, 6th, 9th, 12th day after inoculation, the adding volume was: A : 9%(3rd d) - 9%(6^{th} d) - 9%(9^{th}d) - 9%(12^{th} d) ; B : 6%(3rd d) - 12%(6^{th} d) - 12%(9^{th}d) - 6%(12^{th} d) ; C : 12%(3rd d) -12%(6^{th} d) - 6%(9^{th}d) - 6%(12^{th} d) ; D : 6%(3rd d ) - 6%(6^{th} d) - 12%(9^{th}d) - 12%(12^{th} d).

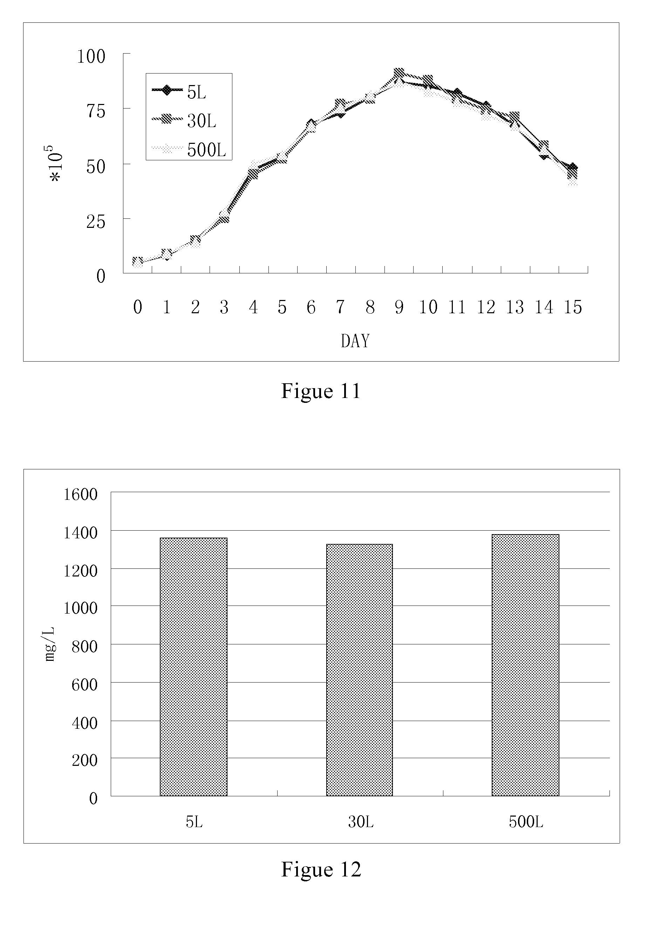

Figure 11: Cell growth curve of different culture scale, cells cultured in 5L, 30L, and 500L bioreactors respectively, 300S was added according to the following scheme: once every three days from 72 hours after inoculation, the adding volume was 6%(3rd d) - 12%(6^{th} d)) - 12%(9^{th}d) - 6%(12^{th} d) of the initial culture volume.
Figure 12: Cell expression results of different culture scale, cells cultured in 5L, 30L, and 500L bioreactors respectively, 300S was added according to the following scheme: from 72 hours after inoculation, once every three days, the adding volume was 6%(3rd d) - 12%(6^{th} d)) - 12%(9^{th}d) - 6%(12^{th} d) of the initial culture volume.

### DESCRIPTION OF PREFERRED EMBODIMENTS:

Through the broad and deep study, the inventors of this patent herein disclosed a medium which comprises high amount of nutrients. Furthermore, the inventors discovered that in the large-scale fed-batch culture process of CHO cells, cell growth may be improved significantly by a certain way by adding the medium,

As used herein, "concentrated medium" and "medium which contains high amount of nutrients" can be used interchangeably. They all refer to the medium disclosed in Chinese patent CN200710085142.2 based on large-scale CHO cell culture, and obtained through adjusting certain concentrations of some components. Herein, the applicant also incorporates in the full text of Chinese patent CN200710085142.2 as reference.

As used herein, "common medium" refers to the medium disclosed in Chinese patent CN200710085142.2 and is used to culture large-scale CHO cells. It comprises:

| | |
|---|---|
| Vitamin C | 10-25mg/L |
| Transferrin | 5 -18mg/L |
| Ethanolamine | 1-10mg/L |
| Selenite | 0.01-0.045 mg/L |
| Hydroxybutyrate sodium | 0.5-1.5mg/L |
| AlCl₃•6H₂O | 0.5-2.5mg/L |
| AgNO₃ | 0.02-0.045mg/L |
| CoCl₃•6H₂O | 0.05-0.3mg/L |
| CuSO₄•5H₂O | 0.002-0.004mg/L |
| KBr | 0.0005-0.0008mg/L |
| FeSO₄•7H₂O | 0.5-1.5mg/L |
| Na₂SiO₃ | 0.005-0.045mg/L |
| LiCl | 0.0005-0.001mg/L |
| NiCl₂•6H₂O | 0.02-0.25 mg/L |
| SnCl₂•2H₂O | 2.00-8.00mg/L |
| ZnSO₄•7H₂O | 0.5-2.5mg/L |
| Vitamin B6 | 0.01-0.2mg/L |
| Vitamin B12 | 0.01-0.5mg/L |
| Biotin | 0.05-0.18mg/L |
| Folic Acid | 0.02-8.00mg/L |
| Ribo flavin | 0.1-0.15mg/L |
| reduced glutathione | 0.5-2.5mg/L |
| Adenine | 5.5-8.5 mg/L |
| Guanine | 5.5-8.5 mg/L |
| Uracil | 5.5-8.5 mg/L |
| Thymine | 0.25-1.5mg/L |
| Cytosine | 5.5-8.5mg/L |
| Ribose | 2.0-8.5mg/L |
| Deoxyribose | 2.0-8.5mg/L |
| Primatone | 500-3500mg/L |
| 4-hydroxyethyl-piperazine ethanesulfonic acid (HEPES) | 500-3000mg/L |
| β-mercaptoethanol | 0.2-2mg/L |

On the basis of the common medium above mentioned, the inventors adjusted the amount of components in the table 1 to get the concentrated medium in this invention.

The preparation method is the same as that disclosed in Chinese patent CN200710085142.2. Adjusted the PH to 6.8-7.2 after preparation, Osmotic pressure is between 300 - 1000mOsm/kg.

**Table 1 the components adjusted and the amount in concentrated medium**

| Components | amount (mg/L) | preferably amount (mg/L) | Optimal amount (mg/L) |
|---|---|---|---|
| Sodium chloride | 1000-9000 | 1500-6599 | 2500-4500 |
| Aspartate | 200-4000 | 350-2000 | 500-1250 |
| Asparagine | 200-9000 | 550-5000 | 1000-4000 |
| Glutamate | 100-5000 | 250-3000 | 500-2000 |
| Isoleucine | 200-3000 | 500-2500 | 1000-2000 |
| Leucine | 300-4500 | 500-2500 | 750-1500 |
| Methionine | 50-1000 | 80-700 | 150-450 |
| Phenylalanine | 100-2000 | 200-1250 | 300-750 |
| Serine | 500-10000 | 2000-7000 | 3500-6000 |
| Threonine | 50-1500 | 100-700 | 150-450 |
| Tryptophan | 25-1000 | 80-350 | 125-200 |
| Valine | 40-1000 | 80-400 | 120-300 |
| Arginine | 150-2000 | 300-1200 | 350-600 |
| Biotin | 0.01-0.5 | 0.05-0.2 | 0.075-0.15 |
| Calcium Pantothenate | 2-100 | 10-70 | 20-50 |
| Choline chloride | 3-270 | 10-90 | 30-60 |
| Folic Acid | 1-30 | 2-15 | 3-7 |
| Inositol | 5-900 | 25-500 | 75-350 |
| Nicotinic acid amine | 0.1-10 | 0.5-5 | 1-3 |
| Riboflavin | 0.01-1 | 0.1-0.5 | 0.15-0.3 |
| Thiamine | 1-100 | 5-50 | 10-30 |
| Vitamin B6 | 0.01-5 | 0.1-2 | 0.2-1.0 |

The process of the cell culture is also controlled and affected by inoculated cell density, the adding method and the time of adding the concentrated medium, pH value, glucose concentration and ventilation factors etc.

### 1 . The effect of inoculated cell density

The starting density is very important for cell culture on large-scale. Too high initial density will lead to insufficient amplification space during the culture process, barriers in the supply of nutrients and oxygen in medium, serious metabolic waste accumulation, decreased cell survival rate in advance, short culture cycles and lower expression. Too low density may lead to inadequate cell number, longer culture cycles, lower density and lower expression in the following culturing process. In our research, we found that 2×10⁵/ml - 2×10⁶/ml density is the suitable density, in particular 3×10⁵/ml - 1×10⁶/ml, preferably the initial density is 5×10⁵/ml. With the appropriate density, at 4-7 days after inoculation, cells density reached to the highest point about 6×10⁶/ml - 1×10⁷/ml , when the viable cell rate was about 90 - 95%, and the cells may continue to be amplified or change into batch culture.

### 2. The adding method and time of adding the concentrated medium

When the concentration of the cells reached to 1 - 5×10⁶/ml at 1-5days after cell inoculation, begin to add the concentrated medium for several times. The total volume added is 10% - 100% of the initial culture volume, in particular, 15% - 60%, and the best supplementary volume is 30% - 40% of initial culture volume.

The supplementary frequency of concentration medium is once or twice every day to once every five days. And the best frequency of supplement is once in three days. The supplement volume of concentration medium can be the same or different each time.

The supplement period of the concentration medium is about 12 days. The volume of the supplement of fluid in the first and last three days is less, it is 30% - 50% of the total volume of the supplement volume. The volume of the supplement of fluid in the middle six days is 50% - 70% of the total volume. Optimized fluid distribution is 17% - 64% - 17%(the first three days-intermediate six days-the last three days).

### 3. pH control

The pH control is very important to the process of culture. The suitable pH is between 6.5 and 7.5, in particular between 6.7 and 7.3. The most favorable pH is between 6.8 and 7.1. In the first several days after inoculation, the pH was set between 6.9 and 7.1. The pH value was adjusted by supplementing CO₂ or NaHCO₃. Once the concentrated medium began to add, pH value was set between 6.8 and 7.0 in the process of cell culture. In the late period of culture, the pH value was sat back between 6.9 and 7.1.

### 4. Glucose control

Glucose concentration is very important to the process of batch culture. Too lower glucose concentration may cause cell death due to "starvation", but too higher glucose concentration may inhibit cells growth, increase osmotic pressure and result in excessive lactic acid production. The glucose concentration may keep a suitable range by supplementing 20% - 50% of glucose solution every day. The suitable glucose concentration is 0.5-10g/L, in particular 1 - 5g/L , the most suitable concentration is 2.5 - 3.5g/L.

### 5. Adjustment of ventilation

Bubble-free or micro-bubble ventilation may be used in the process of culturing. The gases for ventilation include air, oxygen and carbon dioxide. Bioreactor automatically adjusts the ratio of air with oxygen to maintain the dissolved oxygen around the set value. Carbon dioxide is used to control the pH of bioreactor. In the bubble-free ventilation mode, ventilation is controlled between 0.5-2vvm(vessel volume per minute). In the micro-bubble ventilation mode, ventilation is controlled between 0.001-0.01vvm. Under the condition of effective control of pH and dissolved oxygen, ventilation should be as low as possible.

The parameter mentioned above, or mentioned in embodiment in this invention may be combined unconventionally. All the features revealed in the description can be co-used with the composition form; every feature revealed in the description can be substituted by any same, equal or similar alternative feature. Therefore, unless there is a specific specification, the revealed equal or similar feature is only the general case.

The main advantages of this invention are:
1 Discovered a kind of formulation of a concentrated medium which may be used to culture CHO cell on large-scale.
2 The concentrated medium disclosed in this invention was applied to the process of Fed-batch CHO cells culture on large-scale.

Hereafter the invention was illustrated by embodiments. These embodiments are used to illustrate the invention only, but do not limit the scope of the invention. For the below embodiments, method that not indicate the specific experimental means that it is in accordance with the common conditions or the conditions recommended by the manufacturer generally. Unless it is specifically stated, all the percentage and portions are all measured by weight.

Unless otherwise defined, the meaning of all the professional and scientific terms used herein is the same with the meaning mastered by the skilled personnel in this area. In addition, any recorded similar contents or equal methods and materials can be applied in this invention. The mentioned optimal embodiments and materials are for demonstration purposes only.

### Example 1:

### The preparation of 300F medium and 4 kinds of concentrated medium

300F was acquired by adding the following substances to basic medium, DMEM/F12 medium (purchased from Sigma company), all calculated according to the final volume of medium CHO cells

| | |
|---|---|
| Vitamin C | 10.5 mg/L ; |
| Transferrin | 6.2mg/L; |
| Ethanolamine | 3.1mg/L; |
| Selenite | 0.025mg/L; |
| Hydroxybutyrate Sodium | 0.85mg/L, |

### Trace elements :

| | |
|---|---|
| AlCl₃·6H₂O | 1.2 mg/L |
| AgNO₃ | 0.03 mg/L |
| CoCl₂·6H₂O | 0.21 mg/L |
| CuSO₄·5H₂O | 0.0027mg/L |
| KBr | 0.00061 mg/L |
| FeSO₄·7H2O | 0.72 mg/L |
| Na₂SiO₃ | 0.005-0.045 mg/L |
| LiCl | 0.00083 mg/L |
| NiCl₂·6H₂O | 0.042 mg/L |
| SnCl₂·2H₂O | 2.12 mg/L |
| ZnSO₄·7H₂O | 1.58 mg/L |
| Vitamin : | |
| Vitamin B6 | 0.18 mg/L |
| Vitamin B12 | 0.38 mg/L |
| Biotin | 0.12 mg/L |
| Folic acid | 3.12 mg/L |
| Riboflavin | 0.12 mg/L |

And the following substances:

| | |
|---|---|
| reduced Glutathione | 0.68 mg/L |
| Adenine | 6.5 mg/L |
| Guanine | 6.5mg/L |
| Uracil | 6.5 mg/L |
| Thymine | 0.7 mg/L |
| Cytosine | 6.5 mg/L |
| Ribose | 2.0 mg/L |
| Deoxyribose | 2.0mg/L |
| HEPES(4-hydroxyethyl-piperazine ethanesulfonic acid) | 2100 mg/L |
| β-mercaptoethanol | 1.5mg/L |

On the basis of 300F, the following components were adjusted. The detailed amount showed in table2, 4 kinds of concentrated medium were got and named as 3005 , 300S1 , 300S2 and 300S3 respectively. The preparation process as follows: dissolved the powder with injected water of 80% - 90% of the final volume, stirred for 3-4 hours, the pH was measured, adjusted to 6.8-7.2 with 1mol/L sodium hydroxide or 1mol/L hydrochloric acid, then added the injection water to final volume.

**Table 2 components adjusted and their amount of the 4 kinds of concentrated medium**

| components | Amount 300S (mg/L) | Amount 300S1 (mg/L) | Amount 300S2 (mg/L) | Amount 300S3 (mg/L) |
|---|---|---|---|---|
| Sodium chloride | 3000 | 1500 | 6599 | 2500 |
| Aspartate | 800 | 350 | 2000 | 500 |
| Asparagine | 2000 | 550 | 5000 | 4000 |
| Glutamate | 1200 | 250 | 3000 | 2000 |
| Isoleucine | 1500 | 500 | 500 | 1000 |
| Leucine | 1250 | 500 | 500 | 750 |
| Methionine | 350 | 80 | 700 | 450 |
| Phenylalaninee | 450 | 200 | 1250 | 300 |
| Serine | 5000 | 2000 | 7000 | 3500 |
| Threonine | 300 | 700 | 100 | 450 |
| Tryptophan | 150 | 350 | 80 | 125 |
| Valine | 200 | 400 | 400 | 300 |
| Arginine | 450 | 300 | 1200 | 600 |
| Biotin | 0.1 | 0.2 | 0.05 | 0.075 |
| Calcium Pantothenate | 35 | 10 | 70 | 50 |
| Choline chloride | 30 | 90 | 10 | 30 |
| Folic Acid | 4 | 15 | 2 | 7 |
| Inositol | 200 | 25 | 500 | 75 |
| Nicotinic acid amine | 2 | 0.5 | 5 | 1 |
| Riboflavin | 0.2 | 0.1 | 0.5 | 0.15 |
| Thiamine | 20 | 50 | 5 | 30 |
| Vitamin B6 | 0.5 | 0.1 | 2 | 1 |

### Example 2

### The effect on cell growth and cell expression after supplementary 300S, 300S1, 300S2 or 300S3

Cell : if no specification, the cell in the examples is selected from sTNFR fusion gene transfected CHO cell (the preparation method disclosed in Chinese Patent 01132074.5 ), the same below.

When grown to logarithmic phase in seed bioreactor, 5×10⁵/ml cells were inoculated to 5L bioreactor with 300F Medium. The fed-batch cell culture process started with 60% of vessel work volume as initial volume. The ventilation mode was bubble-free during the cell culture process. PH was controlled between 6.8-7.1 during cell culture by adding 7.5% Sodium bicarbonate or carbon dioxide. Measure the residual sugar concentration in medium once everyday, and supplemented 30% glucose to keep the concentration of residual sugar in bioreactor between 2.5-3.5g/L. Dissolved oxygen was controlled between 40 - 60% adjusted by oxygen and compressed air. Six kind of fed programs were proposed: first, added glucose and alkaline solution when needed only, no other nutritious components added; second, 72 hours after inoculation, added 300F daily, the fed volume was 2% of the initial culture volume. Third, 72 hours after inoculation, added 300S daily, the feed volume was 2% of the initial culture volume. Fourth, 72 hours after inoculation, added 300S1 daily, the fed volume was 2% of the initial culture volume. Fifth, 72 hours after inoculation, added 300S2 daily, the fed volume was 2% of the initial culture volume. Sixth, 72 hours after inoculation, added 300S3 daily, the fed volume was 2% of the initial culture volume. Viable cells were counted everyday. The end point of culture process was set on the 15th day of cell culture or when the viable cells density was less than 10×10⁵/ml. Supernatants of cell culture was harvested and the concentration of interesting protein was assayed by ELISA.

Result: as shown in figure 1, without adding any medium, cells couldn't maintain to the 15th day. After adding 300F, cells could maintain to the 15th day but couldn't reach high density. While after adding 300S , 300S1 300S2 and 300S3 , the density of viable cells increased obviously, and duration time was significantly prolonged, and at the meantime, the best result was acquired by adding 300S. Figure 2 shows the cells' expression results after adding 300S 300S1 , 300S2 , 300S3, and 300S was most suitable to cell fed-batch culture process.

### Example 3

### Comparison of the different amount of 300S on cell growth and expression

When grown to logarithmic phase in seed bioreactor, 5×10⁵/ml cells were inoculated to 5L bioreactor with 300F Medium. The fed-batch cell culture process started with 60% of vessel work volume as initial volume. The ventilation mode was bubble-free during the cell culture process. PH was controlled between 6.8-7.1 during cell culture by adding 7.5% Sodium bicarbonate or carbon dioxide. Measured the residual sugar concentration in medium daily, and supplemented 30% glucose to keep the concentration of residual sugar in bioreactor between 2.5-3.5g/L. Dissolved oxygen was controlled between 40% - 60% adjusted by oxygen and compressed air.72 hours after inoculation, 300S was added daily, the fed volume was 1% , 2% , 3% , 4% and 5% of initial culture volume respectively. Viable cells were counted everyday. The end point of culture process was set on the 15th day of cell culture or when the viable cells density was less than 10×10⁵/ml. Supernatants of cell culture was harvested and the concentration of interesting protein was assayed by ELISA.

Result: as shown in figure 3, the highest viable cell density was acquired in bioreactor added 300S 3%. Figure 4 also shows that the highest expression level was acquired in the same bioreactor. Therefore, adding 300S 3% of the initial cell culture volume daily was most beneficial fed regimen to fed-batch cell culture process.

### Example 4

### The relation between the time on initial time of supplement 300S and cell growth and expression

When grown to logarithmic phase in seed bioreactor, 5×10⁵/ml cells were inoculated to 5L bioreactor with 300F Medium. The fed-batch cell culture process started with 60% of vessel work volume as initial volume. The ventilation mode was bubble-free during the cell culture process. PH was controlled between 6.8-7.1 during cell culture by adding 7.5% Sodium bicarbonate or carbon dioxide. Measure the residual sugar concentration in medium daily, and supplemented 30% glucose to keep the concentration of residual sugar in bioreactor between 2.5-3.5g/L. Dissolved oxygen was controlled between 40 - 60% adjusted by oxygen and compressed air. From the 1st, 2nd, 3rd, 4th or 5th day after inoculation, 300S was added daily, the fed volume was 3% of initial culture volume respectively. Viable cells were counted everyday. The end point of culture process was set on the 15th day of cell culture or when the viable cells density was less than 10×10⁵/ml. Obtained supernatants of cell culture was harvested and the concentration of interesting protein was assayed by ELISA.

Result: as shown in figure 5, the highest viable cell density was acquired in bioreactor fed 300S from the third day. Figure 6 also shows that the highest expression level was acquired in the same bioreactor. Therefore, the third day after innoculation is the most suitable start point for medium fed, and it was most beneficial to fed-batch cell culture process.

### Example 5

### The relation between the interval time on supplement 300 S and cell growth and expression

When grown to logarithmic phase in seed bioreactor, 5×10⁵/ml cells were inoculated to 5L bioreactor with 300F Medium. The fed-batch cell culture process started with 60% of vessel work volume as initial volume. The ventilation mode was bubble-free during the cell culture process. PH was controlled between 6.8-7.1 during cell culture by adding 7.5% Sodium bicarbonate or carbon dioxide. Measure the residual sugar concentration in medium daily, and supplemented 30% glucose to keep the concentration of residual sugar in bioreactor between 2.5-3.5g/L. Dissolved oxygen was controlled between 40 - 60% adjusted by oxygen and compressed air.300S began to feed at72 hours after inoculation, There were five fed regimen: first, once fed every 12 hours, each bolus was 1.5% of initial culture volume. Second, once fed per day, each bolus was 3% of initial culture volume. Third, once fed every two days, each bolus was 6% of initial culture volume. Fourth, once fed every three days, each bolus was 9% of initial culture volume. Fifth, once fed every four days, each bolus was 12% of initial culture volume. Viable cells were counted everyday. The end point of culture process was set on the 15th day of cell culture or when the viable cells density was less than 10×10⁵/ml. Supernatant of cell culture was harvested and the concentration of interesting protein was assayed by ELISA.

Result: as shown in figure 7, fed 300S after 72 hours, the densities were quite close to each other under these four regimens: twice fed per day, once a day, once every two days and once every three days. Figure 8 also shows that the expression levels have little difference. And only in the regimen of once fed every four days, the cell density decreased obviously and the expression level was lower significantly. Simple operation is the best. Therefore, from 72 hours after inoculation, feeding 300S once every three days is the best choice.

### Example 6

### The relation between different supplement schemes of 300S and cell growth and expression

When grown to logarithmic phase in seed bioreactor, 5×10⁵/ml cells were inoculated to 5L bioreactor with 300F medium. The fed-batch cell culture process started with 60% of vessel work volume as initial volume. The ventilation mode was bubble-free during the cell culture process. PH was controlled between 6.8-7.1 during cell culture by adding 7.5% Sodium bicarbonate or carbon dioxide. Measure the residual sugar concentration in medium once everyday, and supplemented 30% glucose to keep the concentration of residual sugar in bioreactor between 2.5-3.5g/L. Dissolved oxygen was controlled between 40 - 60% adjusted by oxygen and compressed air. At 72 hours after inoculation, 300S was begun to add once every three days, the total fed volume was the same: 36% of initial culture volume. There were four supplement regimens, as shown in table 3. Viable cells were counted everyday. The end point of culture process was set on the 15th day of cell culture or when the viable cells density was less than 1×10⁵/ml. Supernatant of cell culture was harvested and the concentration of interesting protein was assayed by ELISA.

**Table 3 four supplement regimens of 300S**

| Addition time (n day after inoculation ) | 3^{rd} day | 6^{th} day | 9^{th} day | 12^{th} day |
|---|---|---|---|---|
| scheme 1 | 9% | 9% | 9% | 9% |
| scheme 2 | 6% | 12% | 12% | 6% |
| scheme 3 | 12% | 12% | 6% | 6% |
| scheme 4 | 6% | 6% | 12% | 12% |

Result: as shown in figure 9, supplement of 300S started from 72 hours after inoculation, once fed every three days, the viable cell densities were close in regimen 1 and regimen 2. The densities of regimen 3 and regimen 4 were comparatively lower. Figure 10 showed that scheme 2 acquired the highest expression level.

### Example 7

### The enlargement of cell culture process

To further verify the effect of 300S to CHO cells, we used bioreactors with different sizes ,mainly 5L 30L and 500L, to repeat the processes mentioned above. When grown to logarithmic phase in seed bioreactor, 5×10⁵/ml cells were inoculated to bioreactors with 300F Medium. The fed-batch cell culture process started with 60% of vessel work volume as initial volume. The ventilation mode was bubble-free in 5L bioreactor and microbubble in 30L and 500L bioreactors during the cell culture process. PH was controlled between 6.8-7.1 during cell culture by adding 7.5% Sodium bicarbonate or carbon dioxide. Measure the residual sugar concentration in medium daily, and supplemented 30% glucose to keep the concentration of residual sugar in bioreactor between 2.5-3.5g/L. Dissolved oxygen was controlled between 40% - 60% adjusted by oxygen and compressed air. From 72 hours after inoculation, 300S was started to feed once every three days, the supplement volume on the 3rd day was 6% of the initial culture volume, the supplement volume on 6th and 9th day was 12% of the initial culture volume, and the supplement volume on 12th day was 6% of the initial culture volume. Viable cells were counted everyday. The end point of culture process was set on the 15th day of cell culture or when the viable cells density was less than 10× 10⁵/ml. Supernatant of cell culture was harvested and the concentration of interesting protein was assayed by ELISA.

Result: as shown in figure 11 and figure 12, growth curves and expression levels were similar on different cell culture scales and different ventilation modes, so the concentrated medium and supplement regimen could be enlarged to pilot scale or applied on production scale.

This medium also be tested on another two cells: CHO cell that express sCTLA4 fusion protein and CHO cell that express humanized anti-HER2 monoclonal antibody( according to the preparation method disclosed in the examples in Chinese Patent 01132075.3 and 0113225.X separately), and concentrated medium named 300S-1( substitute 300S for culturing CHO cells of expression sCTLA4 fusion protein) and 300S-2( substitute 300S for culturing CHO cell of expression humanized anti-HER2 monoclonal antibody) was prepared according to the metabolic characteristics of these 2 kinds of cells, separately, see in table 4. Then, 300S-1 and 300S-2 were used to replace 300S respectively, according to the method in example 2-7, and the similar results were obtained. It meant that the concentrated medium and its supplement method in this invention also could be applied to other CHO cells on pilot or production scales.

**Table 4 components adjusted and their amount in the concentrated medium**

| Component | 300S-1 amount (mg/L) | 300S-2 amount (mg/L) |
|---|---|---|
| Sodium chloride | 3000 | 3000 |
| Aspartate | 600 | 1000 |
| Asparagine | 1500 | 2500 |
| Glutamate | 800 | 800 |
| Isoleucine | 1200 | 1500 |
| Leucine | 900 | 900 |
| Methionine | 450 | 450 |
| Phenylalanine | 450 | 375 |
| Serine | 3500 | 4500 |
| Threonine | 450 | 300 |
| Tryptophan | 250 | 100 |
| Valine | 250 | 150 |
| Arginine | 450 | 450 |
| Biotin | 0.1 | 0.1 |
| Calcium Pantothenate | 25 | 50 |
| Choline chloride | 120 | 70 |
| Folic Acid | 8 | 4 |
| Inositol | 120 | 150 |
| Nicotinic acid amine | 1.5 | 2 |
| Riboflavin | 0.2 | 0.15 |
| Thiamine | 24 | 12 |
| Vitamin B6 | 0.25 | 0.25 |

No matter to the common medium used in the process of cell culture or to the concentrated medium needed to supplement in the process of fed-batch culture, the changes on its composition components and the changes of their concentration component are related determined by the specific cell lines cultured. The common medium and/or the concentrated medium which is prepared for particular cell lines have much better effect to culture of the cell line, such as 300S for CHO cells which express sTNFR fusion protein, 3005-1 for CHO cells which express r expression sCTLA4 fusion protein, and 300-2 for CHO cells which express humanized anti-HER2 monoclonal antibody in the abovementioned examples. When the concentrated medium mentioned in this invention is used to culture common cell lines, the cell growth may also be improved and enhanced, for it contains components that are essential to the normal cell growth.

All the documents mentioned in this invention are quoted by the application as reference, just as every document be quoted alone as reference. In addition, it should be understood that after reading the content of the invention, technician in this field may change or modify the invention. These equivalent forms are also included in the scope of the claims in this application attached.

## Claims

1. A kind of concentrated medium suitable for Chinese Hamster Ovary cells culture on large-scale, comprises basic medium and additives, wherein according to the whole volume of the concentrated medium, the additives are:
| | |
|---|---|
| Vitamin C | 10-25mg/L |
| Transferrin | 5-18mg/L |
| Ethanolamine | 1-10mg/L |
| Selenite | 0.01-0.045 mg/L |
| Hydroxybutyrate sodium | 0.5-1.5mg/L |
| AlCl₃•6H₂O | 0.5-2.5mg/L |
| AgNO₃ | 0.02-0.045mg/L |
| CoCl₃•6H₂O | 0.05 -0.3mg/L |
| CuSO₄•5H₂O | 0.002-0.004mg/L |
| KBr | 0.0005-0.0008mg/L |
| FeSO₄•7H₂O | 0.5-1.5mg/L |
| Na₂SiO₃ | 0.005-0.045mg/L |
| LiCl | 0.0005-0.001mg/L |
| NiCl₂•6H₂O | 0.02-0.25 mg/L |
| SnCl₂•2H₂O | 2.00-8.00mg/L |
| ZnSO₄•7H₂O | 0.5-2.5mg/L |
| Vitamin B6 | 0.01-0.2mg/L |
| Vitamin B12 | 0.01-0.5mg/L |
| Biotin | 0.05-0.18mg/L |
| Folic Acid | 0.02-8.00mg/L |
| Ribo flavin | 0.1-0.15mg/L |
| Reduced glutathione | 0.5-2.5mg/L |
| Adenine | 5.5-8.5 mg/L |
| Guanine | 5.5-8.5 mg/L |
| Uracil | 5.5-8.5 mg/L |
| Thymine | 0.25-1.5mg/L |
| Cytosine | 5.5-8.5mg/L |
| Ribose | 2.0-8.5mg/L |
| Deoxyribose | 2.0-8.5mg/L |
| Primatone | 500-3500mg/L |
| 4-hydroxyethyl-piperazine ethanesulfonic acid (HEPES) | 500-3000mg/L |
| β-mercaptoethanol | 0.2-2mg/L, |
**characterised in that** concentrations of the following components of in the said concentrated medium are as below:
| | |
|---|---|
| 1000-9000mg/L | Sodium chloride |
| 200-4000 mg/L | Aspartate |
| 200-9000 mg/L | Asparagine |
| 100-5000 mg/L | Glutamate |
| 200-3000 mg/L | Isoleucine |
| 300-4500 mg/L | Leucine |
| 50-1000 mg/L | Methionine |
| 100-2000 mg/L | Phenylalanine |
| 500-10000 mg/L | Serine |
| 50-1500 mg/L | Threonine |
| 25 -1000 mg/L | Tryptophan |
| 40-1000 mg/L | Valine |
| 150-2000 mg/L | Arginine |
| 0.01-0.5 mg/L | Biotin |
| 2-100 mg/L | Calcium Pantothenate |
| 3-270 mg/L | Choline chloride |
| 1-30 mg/L | Folic Acid |
| 5-900 mg/L | Inositol |
| 0.1-10 mg/L | Nicotinic acid amine |
| 0.01-1 mg/L | Riboflavin |
| 1-100 mg/L | Thiamine |
| 0.01-5 mg/L | Vitamin B6 |

2. A kind of concentrated medium according to claim 1, **characterised in that** concentrations of the following components of the concentrated medium are as below:
| | |
|---|---|
| 1500-6599 mg/L | Sodium chloride |
| 350-2000 mg/L | Aspartate |
| 550-5000 mg/L | Asparagine |
| 250-3000 mg/L | Glutamate |
| 500-2500 mg/L | Isoleucine |
| 500-2500 mg/L | Leucine |
| 80-700 mg/L | Methionine |
| 200-1250 mg/L | Phenylalanine |
| 2000-7000 mg/L | Serine |
| 100-700 mg/L | Threonine |
| 80-350 mg/L | Tryptophan |
| 80-400 mg/L | Valine |
| 300-1200 mg/L | Arginine |
| 0.05-0.2 mg/L | Biotin |
| 10-70 mg/L | Calcium Pantothenate |
| 10-120 mg/L | Choline chloride |
| 2-15 mg/L | Folic Acid |
| 25-500 mg/L | Inositol |
| 0.5-5 mg/L | Nicotinic acid amine |
| 0.1-0.5 mg/L | Ribo flavin |
| 5-50 mg/L | Thiamine |
| 0.1-2 mg/L | Vitamin B6 |

3. A preparation method of the concentrated medium according to claim 1, wherein the said preparation method comprises following step: mix the basic medium with additives, the said additives comprises the components as below according to the total volume of the concentrated medium :
| | |
|---|---|
| Vitamin C | 10-25mg/L |
| Transferrin | 5-18mg/L |
| Ethanolamine | 1-1 0mg/L |
| Selenite | 0.01-0.045 mg/L |
| Hydroxybutyrate sodium | 0.5-1.5mg/L |
| AlCl₃•6H₂O | 0.5-2.5mg/L |
| AgNO₃ | 0.02-0.045mg/L |
| CoCl₃•6H₂O | 0.05-0.3mg/L |
| CuSO₄•5H₂O | 0.002-0.004mg/L |
| KBr | 0.0005-0.0008mg/L |
| FeSO₄•7H₂O | 0.5-1.5mg/L |
| Na₂SiO₃ | 0.005-0.045mg/L |
| LiCl | 0.0005-0.001mg/L |
| NiCl₂•6H₂O | 0.02-0.25 mg/L |
| SnCl₂•2H₂O | 2.00-8.00mg/L |
| ZnSO₄•7H₂O | 0.5-2.5mg/L |
| Vitamin B6 | 0.01-0.2mg/L |
| Vitamin B12 | 0.01-0.5mg/L |
| bioton | 0.05-0.18mg/L |
| folic acid | 0.02-8.00mg/L |
| ribo flavin | 0.1-0.15mg/L |
| reduced glutathione | 0.5-2.5mg/L |
| adenine | 5.5-8.5 mg/L |
| guanine | 5.5-8.5 mg/L |
| uracil | 5.5-8.5 mg/L |
| thymine | 0.25-1.5mg/L |
| cytosine | 5.5-8.5mg/L |
| ribose | 2.0-8.5mg/L |
| deoxyribose | 2.0-8.5mg/L |
| primatone | 500-3500mg/L |
| 4-hydroxyethyl-piperazine | 500-3000mg/L |
| ethanesulfonic acid (HERPES) | |
| β-mercaptoethanol | 0.2-2mg/L, |
**characterised in that** concentrations of the following components adjusted as below:
| | |
|---|---|
| 1000-9000mg/L | Sodium chloride |
| 200-4000 mg/L | Aspartate |
| 200-9000 mg/L | Asparagine |
| 100-5000 mg/L | Glutamate |
| 200-3000 mg/L | Isoleucine |
| 300-4500 mg/L | Leucine |
| 50-1000 mg/L | Methionine |
| 100-2000 mg/L | Phenylalanine |
| 500-10000 mg/L | Serine |
| 50-1500 mg/L | Threonine |
| 25-1000 mg/L | Tryptophan |
| 40-1000 mg/L | Valine |
| 150-2000 mg/L | Arginine |
| 0.01-0.5 mg/L | Biotin |
| 2-100 mg/L | Calcium Pantothenate |
| 3-270 mg/L | Choline chloride |
| 1-30 mg/L | Folic Acid |
| 5-900 mg/L | Inositol |
| 0.1-10 mg/L | Nicotinic acid amine |
| 0.01-1 mg/L | Riboflavin |
| 1-100 mg/L | Thiamine |
| And 0.01-5 mg/L | Vitamin B6 |
preferably ,the said basic medium is Dulbecco's Modified Eagle's medium (DMEM ) .

4. The usage of the concentrated medium according to claim 1,**characterised in that** the said usage comprises steps of: at 1-5 days after cell inoculation, adding the concentrated medium mentioned in claims 1, the supplement volume is 10% - 100% of the initial culture volume, the said cell is Chinese Hamster Ovary cell.

5. The usage according to claim 4, **characterised in that** the cells inoculation density of inoculation is 2×10⁵/ml - 2×10⁶/ml.

6. The usage according to claim 4, **characterised in that** the said Chinese Hamster Ovarian Ovary Cell Line is selected from CHO cells that express sTNFR fusion protein, CHO cells that express sCTLA4 fusion protein, or CHO cells that express humanized anti-HER2 monoclonal antibody.

7. The usage according to claim 4, **characterised in that** the addition amount supplement volume is 15% - 60% of initial culture volume.

8. The usage according to claim 4, **characterised in that** supplement the concentrated medium 1-5 times daily or once every 1-5 days ,preferably adding the concentrated medium 2-4 times daily or once in every 2-4 days.

9. The usage according to claim 4, **characterised in that** the time for supplement concentrated medium lasting for 5-20 days , preferably lasting for 8-16 days.

10. The usage according to claim 4, **characterised in that** adding the same volume of concentrated medium each time or adding 15% - 25% of the concentrated medium needed by volume in the first 5 days, adding 50% - 70% of the concentrated medium needed by volume in the intermediate 3-9 and adding 15% - 25% of the concentrated medium needed by volume the last 1-5 days.
